# EUROPEAN PATENT APPLICATION

(11) **EP 4 248 969 A1**
(43) Date of publication of application: **27.09.2023**
(21) Application number: 22305343.0
(22) Date of filing: 23.03.2022
(51) Int. Cl.: A61K 31/4453, A61P 25/00

(54) **USE OF PITOLISANT FOR TREATING SEVERE FATIGUE**

(71) Applicant: Bioprojet, 75003 Paris (FR)
(72) Inventor: SCHWARTZ, Jean Charles, 75014 PARIS (FR); LECOMTE, Jeanne-Marie, 75003 PARIS (FR)
(74) Representative: Lavoix

(57) **Abstract**

The present invention concerns the treatment of severe fatigue (SF), in particular in patients suffering from narcolepsy, comprising the administration of pitolisant or its pharmaceutically acceptable salts, such as the monohydrochloride salt at high doses.

## Description

The present invention concerns the therapeutical treatment of severe fatigue.

Severe fatigue (SF) is defined as a subjective experience of mental or physical exhaustion that does not disappear after a period of sleep (Vercoulen et al J. Psychosom. Res. 1994, 38, 383-392).

SF has been found in high percentages of patients with chronic neurological conditions such as Parkinson's disease (Friedman et al., Mov. Disorder, 2007, 22, 297-308), multiple sclerosis (van der Werf et al., J. Neurol. Sci., 1998, 160, 164-170) and neuromuscular disorders (Kalkman et al., J. Psychosom. Res., 2007, 62, 571-579). In these diseases, it was shown that severe fatigue has important clinical consequences, such as increased functional limitations and lowered general health status (Kalkman et al., J. Neurol. Neurosurg. Psychiatry, 2005, 76, 1406-1409).

SF may also be associated with narcolepsy.

The core symptoms of narcolepsy include Excessive Daytime Sleepiness (EDS) and cataplexy. EDS is defined as the inability to stay awake and alert during the major waking episodes of the day, resulting in unintended lapses into drowsiness and/or sleep (Droogleever Fortuyn et al J. Sleep Res. 21, 2, 2012, 163-169).

Although patients may have trouble distinguishing between daytime sleepiness with sleep attacks and the physical feeling of fatigue, excessive daytime sleepiness and fatigue are distinct symptoms that have been shown to co-occur independently in sleep-disordered patients (Lichstein et al Behav. Res. Ther. 1997, 35, 733-740; Merkelbach et al J Sleep Res. 2006 Mar;15(1):105-6).

Droogleever Fortuyn et al, 2011 also reported that SF is highly prevalent in patients with narcolepsy but should be separated from EDS.

As such, the treatment of EDS does not necessarily improves the SF condition.

This is illustrated for example by a meta-analysis of clinical trials of the psychostimulant modafinil in a variety of neurological conditions: it was concluded that the drug was generally effective on EDS without clear effect on fatigue (Sheng et al PLoS One. 2013 Dec 3;8(12):e81802).

A similar conclusion was reached in a trial in traumatic brain injury (Kaiser et al. Neurology. 2010 Nov 16;75(20):1780-5).

Altogether, a large variety of data show that while SF and EDS often co-exist in several neurologic diseases, they are clearly distinct pathological manifestations.

In a recent validation study of the Fatigue Severity Scale (FSS), one of the most currently used fatigue scale, in a cohort study of 429 sleep-wake-disordered patients, Valko et al Sleep, 2008, 31, 1601-1607 showed that the subgroup of narcolepsy patients had the highest fatigue scores.

Droogleever Fortuyn et al, 2011 determined the prevalence of SF in a group of 80 patients with a diagnostic of narcolepsy with cataplexy and its relation with excessive daytime sleepiness, psychological distress, functional impairment and quality of life. Fatigue was measured using the Checklist Individual Strength (CIS). In addition psychological distress, including symptoms of depression, functional impairment and quality of life, were assessed. In this group of patients over 60% of them were found to display SF. There were no age or sex differences between patients with and without EF.

Importantly, both fatigued and non-fatigued patients had the same amount of daytime sleepiness (Epworth Sleepiness Score 14.3 ± 4.2 versus 13.1 ± 4.4, P = 0.22), confirming the separation between sleepiness and fatigue. Severe fatigue was associated with a significantly increased functional impairment, increased depressive symptoms and a lowered general quality of life.

In conclusion, a majority of patients with narcolepsy suffer from severe fatigue, which can be distinguished from daytime sleepiness, and results in severe functional impairment.

In spite of many trials no clear evidence has so far arisen regarding pharmacological treatment of SF in narcolepsy nor even in other neurologic pathologies.

For instance in a recent Cochrane meta-analysis of SF in cancer and other chronic diseases Mücke et al (Cochrane Database Syst Rev. 2015(5):CD006788. doi: 10.1002/14651858.CD006788.pub3. PMID: 26026155; PMCID: PMC6483317) concluded " Based on limited evidence, we cannot recommend a specific drug for the treatment of fatigue. Fatigue research in palliative care seems to focus on modafinil and methylphenidate, which may be beneficial for the treatment of fatigue associated with palliative care although further research about their efficacy is needed".

As another example Nourbakhsh et al (Lancet Neurol. 2021, 20(1):38-48) performed a large double-blind placebo-controlled trial of amantadine, modafinil and methylphenidate for SF in multiple sclerosis and concluded "Amantadine, modafinil, and methylphenidate were not superior to placebo in improving multiple sclerosis fatigue and caused more frequent adverse events. The results of this study do not support an indiscriminate use of amantadine, modafinil, or methylphenidate for the treatment of fatigue in multiple sclerosis".

It thus appears that the usual wake-promoting agents typically used to promote wakefulness and reduce EDS are not efficient to treat SF. In particular, no treatment is indicated for SF in patients with narcolepsy, as well as for other neurological pathologies in which the evidence for efficacy of treatments is also lacking.

It is therefore desirable to provide novel, efficient candidates for treating SF.

Pitolisant (3-(4-Chlorophenyl)propyl 3- piperidinopropyl ether) is of formula :

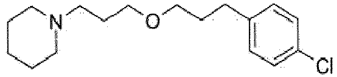

It is a novel histamine H3-receptor antagonist triggering enhanced release of cerebral histamine. Histamine is known as a major wake-promoting neuronal system (Schwartz et al Physiol Rev. 1991 Jan; 71(1):1-51).

The use of pitolisant for treating sleep and vigilance disorders associated with Parkinson's disease, obstructive sleep apnea, narcolepsy, dementia with Lewy bodies and/or vascular dementia, such as EDS is disclosed in WO 2006/103546.

Pitolisant monohydrochloride has been authorized in Europe and in the US and is sold under the brand name Wakix among others, as a medication for the treatment of narcolepsy in adults with or without cataplexy, and excessive daytime sleepiness (EDS) or cataplexy in adult patients with narcolepsy. It represents the first commercially available medication in the class of histamine H3-receptor antagonists/inverse agonists. Recommended doses for treating narcolepsy and/or EDS are comprised between 10 and 40 mg once a day (od).

This dose is in line with studies reporting a high occupancy of the brain histamine H3-receptors at a dose of 40 mg of pitolisant hydrochloride (Rusjan PBr J Pharmacol. 2020 Aug;177(15):3464-3472).

Pitolisant monohydrochloride at doses of 20 to 40 mg, once a day (od) was shown to improve EDS and cataplexy in patients with narcolepsy but its effect on SF was never assessed in these patients suffering from narcolepsy.

In patients with Obstructive Sleep Apnea (OSA), pitolisant monohydrochloride 20 mg od reduced EDS in two trials, whereas SF evaluated with the Pichot scale was statistically different from placebo in one (Dauvilliers Y,et al. Am J Respir Crit Care Med. 2020 May 1;201(9):1135-1145) but not the other (Pepin et al Chest 2021, 159(4), 1598-1609). This further substantiates the conclusion that Fatigue and EDS are distinct entities responding differentially to treatments, and that the use of pitolisant for treating fatigue in OSA patients has not been established so far.

Further, the activity of pitolisant in fatigue associated with other conditions has not been reported.

In tests conducted by the Applicant, it was found that fatigue was not improved by pitolisant at 30 mg dosage in volunteers deprived of sleep during 24h

The effect of pitolisant in fatigue is thus highly unclear.

It is thus desirable to provide a novel treatment of fatigue, in particular fatigue associated with narcolepsy or other conditions.

It has now been identified that pitolisant is active on fatigue, in particular in patients suffering from narcolepsy.

More particularly, it has been discovered that pitolisant is effective at high doses, without adverse effect.

According to a first object, the present invention provides pitolisant for use for treating and/or preventing severe fatigue (SF), comprising administering in a patient suffering from a disorder associated with SF a dose comprised between 50 mg and 240 mg once a day of pitolisant or one of its pharmaceutically acceptable salts, hydrates, or hydrated salts, or the polymorphic crystalline structures of these compounds or their optical isomers, racemates, diastereoisomers or enantiomers.

As used thereafter, "pitolisant" also encompasses its pharmaceutically acceptable salts, hydrates, or hydrated salts, or the polymorphic crystalline structures of these compounds or their optical isomers, racemates, diastereoisomers or enantiomers

As used herein, the terms "severe fatigue", "SF", "excessive fatigue" or "fatigue" are used interchangeably. It refers to the condition characterized by mental or physical exhaustion that does not disappear after a period of sleep (Vercoulen et al J. Psychosom. Res. 1994, 38, 383-392).

There is no available animal model for fatigue.

In human, SF can be measured using a variety of tests, such as the Pichot Fatigue Scale, the Checklist Individual Strength (CIS), or the Flicker Fusion Test.

The CIS is a validated measure with good internal consistency and international recognition. This test can be used to assess SF severity using the Fatigue Severity Scale (FSS) with a score range 8-56. In this scale, a population mean is around 16 and the cut-off for SF is at 35 points (Vercoulen et al, 1994; Droogleever Fortuyn et al, 2011).

The flicker fusion frequency (F.F.F.) is defined as the frequency, in flickers per second, at which an interrupted light appears as a steady light. It has been established that the score made in such tests can be used as a measure of fatigue (Luczak A, et al. Ergonomics, 2005 Dec 15;48(15):1770-92; Ma et al PLoS One, 2014 Feb 5;9(2):e87121; Maeda et al Jpn J Radiol. 2011 Aug;29(7):483-7).

The activity of pitolisant in SF can be assessed by its ability to improve the scores in anyone of the above tests, preferably in the Flicker Fusion Test

As used herein, "pharmaceutically acceptable salts" refer to derivatives of pitolisant which is modified by making acid or base salts thereof. The pharmaceutically acceptable salts include the conventional non-toxic salts or the quaternary ammonium salts of pitolisant formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propanoic, succinic, tartaric, citric, methanesulfonic, benzenesulfonic, glucuronic, glutamic, benzoic, salicylic, toluenesulfonic, oxalic, fumaric, maleic, and the like. Further addition salts include ammonium salts such as tromethamine, meglumine, epolamine, etc., metal salts such as sodium, potassium, calcium, zinc or magnesium. Hydrochloride and oxalate salts are preferred.

The pharmaceutically acceptable salts of the present invention can be synthesized from pitolisant by conventional chemical methods. Generally, such salts can be prepared by reacting the free acid or base forms of pitolisant with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

The monohydrochloride salt of pitolisant is preferred.

According to a further object, the present invention concerns a method of treating and/or preventing severe fatigue (SF), said method comprising administering in a patient in need thereof suffering from a disorder associated with SF a dose comprised between 60 mg and 240 mg once a day of pitolisant or one of its pharmaceutically acceptable salts, hydrates, or hydrated salts, or the polymorphic crystalline structures of these compounds or their optical isomers, racemates, diastereoisomers or enantiomers.

Generally speaking, the identification of those subjects who are in need of treatment of herein-described diseases and conditions is well within the ability and knowledge of one skilled in the art. A clinician skilled in the art can readily identify, by the use of clinical tests, physical examination, genetic tests and medical/family history, those subjects who are in need of such treatment.

According to an embodiment, said patient is suffering from narcolepsy, obstructive sleep apnea (OSA), multiple sclerosis, Parkinson's disease, cancer, depression, bipolar disorder, post stroke disorder, post covid disorder, chronic fatigue syndrome, fibromyalgia, traumatic brain injury, inflammatory bowel disease.

According to a particular embodiment, the patient is not suffering from OSA.

According to a more particular embodiment, the patient is suffering from narcolepsy, such as narcolepsy associated with cataplexy or narcolepsy without cataplexy.

According to the invention, the administration dose between 50 mg and 240 mg once a day refers to the actual dose of pitolisant (ie) in the base form, whereas pitolisant or any of its pharmaceutically acceptable salts, hydrates, or hydrated salts, or the polymorphic crystalline thereof may be administered.

According to an embodiment, the recommended dose of pitolisant may be comprised between 50 and 120 mg and preferably between 60 and 120 mg.

It is also to be understood that this dosage range is meant for adult patients and that equivalent doses in a human child can be adjusted accordingly.

The administration is done once a day (od), preferable in the morning. According to an embodiment, pitolisant is administered upon awakening, before breakfast.

Adjustments of the dosages and/or time of administration can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances. The amount of pitolisant which is required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient and the route of administration.

"Pharmaceutically" or "pharmaceutically acceptable" refers to molecular entities and compositions that do not produce an adverse, allergic or other untoward reaction when administered to an animal, or a human, as appropriate.

In the context of the invention, the term "treating" or "treatment", as used herein, means reversing, alleviating, inhibiting the progress of, or preventing the disorder or condition to which such term applies, or one or more symptoms of such disorder or condition.

"Therapeutically effective amount" means an amount of a compound/medicament according to the present invention effective in producing the desired therapeutic effect.

According to the invention, the term "patient", or "patient in need thereof', is intended for a human or non-human mammal affected or likely to be affected with a neuropsychological disorder. Preferably, the patient is a human.

In general terms, pitolisant may be provided in pharmaceutical compositions with one or more pharmaceutically acceptable excipients.

As used herein, "pharmaceutically acceptable excipient" includes any diluents, adjuvants, or vehicles, such as preserving agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

According to an embodiment, pitolisant may be administered in unit dose forms, wherein the term "unit dose" means a single dose which is capable of being administered to a patient, and which can be readily handled and packaged, remaining as a physically and chemically stable unit dose comprising either pitolisant itself, or as a pharmaceutically acceptable composition, as described hereinafter.

Pitolisant can be formulated into pharmaceutical compositions by admixture with one or more pharmaceutically acceptable excipients.

The compositions may conveniently be administered in unit dosage form and may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition.

According to an embodiment, pitolisant is suitable for oral administration, particularly in the form of tablets.

The tablets, and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate.. In addition, dosage unit forms can contain various other materials that modify the physical form of the dosage unit, for example, coatings of sugar, shellac, or enteric agents. Oral dosage forms syrup or elixir may contain sweetening agents, preservatives, dyes, colorings, and flavorings.

Preferred formulations include pharmaceutical compositions in which pitolisant is formulated for oral administration, and more preferably formulated as a tablet. Oral compositions will generally include an inert diluent carrier or an edible carrier. Preferred tablets contain lactose, cornstarch, magnesium silicate, croscarmellose sodium, povidone, magnesium stearate, or talc in any combination.

### Figures:

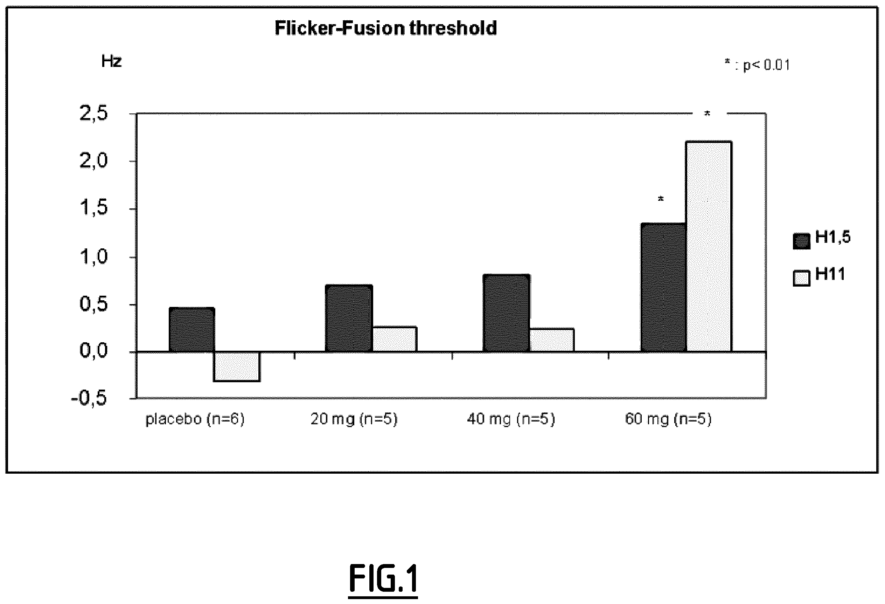

Figure 1 illustrates the absolute difference of Flicker Fusion Threshold (after treatment - before treatment) by treatment group (control or pitolisant monohydrochloride at 20, 40 or 60 mg (* denotes significant change 1.5 or 11h post treatment with pitolisant).

### Examples

### 1. Critical Flicker Fusion Test (CFFT) in healthy volunteers

In human healthy volunteers, fatigue can be evaluated at the end of the day, when fatigue tends to increase, using a known psychometric test, the Flicker Fusion Test as reported by Luczak A, et al. Ergonomics, 2005 Dec 15;48(15):1770-92; Ma et al PLoS One, 2014 Feb 5;9(2):e87121; Maeda et al Jpn J Radiol. 2011 Aug;29(7):483-7.

In a group of 36 healthy volunteers, the change in Fusion Frequency was evaluated at several times along the day and after administration of either placebo or pitolisant monohydrochloride at increasing dosages (20, 40 or 60 mg). The Fusion Frequency was particularly altered at the end of the day, at which time pitolisant monohydrocloride reversed the alteration when given orally at a dose of 60 mg, whereas lower doses failed.to provide a significant change (see Figure 1).

In contrast, in the same group of subjects, wakefulness was assessed by analysis of their Spectral EEG and was found to be already improved significantly at a dose of 40 mg of pitolisant monohydrochloride (not shown).

This illustrates the higher sensitivity to pitolisant of wakefulness compared to Fatigue which requires a higher dosage to be significantly improved.

### 2. In sleep deprived subjects

Pitolisant hydrochloride was administered once a day at a dose of 30 mg in human volunteers deprived of sleep during 24h. Fatigue evaluated in these volunteers by the Fusion Frequency was not modified by either placebo or pitolisant at this dose.

### Conclusion

It thus appears that the Flicker Fusion Test was not improved by a 30 to 40 mg treatment with pitolisant hydrochloride which is known to improve EDS in patients with narcolepsy at these doses.

In contrast, at 60 mg pitolisant hydrochloride administered to tired healthy volunteers, the Flicker Fusion Test was significantly improved.

### 3. Adverse effect

It was found that pitolisant can be used at doses up 240 mg od without overt adverse effect.

A group of 24 healthy volunteers received placebo or pitolisant monohydrochloride mg at doses of 160, 200 or 240 mg. This last dose corresponds to 6 times the maximum dosage recommended in narcolepsy. Blood samples analysed for pitolisant and metabolites level showed dose proportionality. The number and nature of adverse events did not differ in pitolisant- and placebo-treated subjects

No significant abnormalities were recorded on laboratory parameters, vital signs, physical examination and ECG parameters. Furthermore, the rare abnormalities were reported in all treatment groups.

Considering these results, it could be concluded that administration of pitolisant at 160 mg, 200 mg and 240 mg was safe and well tolerated.

### 4. Brain histamine H3 receptors occupancy

6 healthy volunteers receiving 40 mg of pitolisant monohydrochloride. A PET scan study was conducted. The authors reported that the therpautic dose of pitolisant occupied a large fraction of histamine H3 receptors (84 ± 7% ( mean ± SD)) (Rusjan PBr J Pharmacol. 2020 Aug;177(15):3464-3472) and suggested that based on this high occupancy, activity of pitolisant in a variety of CNS disorders associated with H3 receptors may be expected at this therapeutical dose.

This is in line with the significant effect of pitolisant on EDS and cataplexy in patients with narcolepsy at this therapeutical dose.

Accordingly, the lack of improvement of the Flicker Fusion Test at this dose of pitolisant hydrochloride which is known to improve EDS in patients with narcolepsy is thus unexpected.

Also, it could not be expected that a threshold dose of pitolisant hydrochloride higher that this dose already achieving a high occupancy should be required to improve SF.

## Claims

1. Pitolisant for use for treating and/or preventing severe fatigue (SF), comprising administering in a patient suffering from a disorder associated with SF a dose comprised between 50 mg and 240 mg once a day of pitolisant or one of its pharmaceutically acceptable salts, hydrates, or hydrated salts, or the polymorphic crystalline structures of these compounds or their optical isomers, racemates, diastereoisomers or enantiomers.

2. Pitolisant for use according to claim 1 wherein pitolisant is administered at a dose comprised between 50 mg and 120 mg.

3. Pitolisant for use according to anyone of the preceding claims wherein said patient is suffering from narcolepsy, obstructive sleep apnea, multiple sclerosis, Parkinson's diseas, cancer, depression, bipolar disorder, post stroke disorder, post covid disorder, chronic fatigue syndrome, fibromyalgia, traumatic brain injury, inflammatory bowel disease

4. Pitolisant for use according to anyone of the preceding claims, wherein the patient is suffering from narcolepsy.

5. Pitolisant for use according to claim 4 where said patient suffers from narcolepsy associated with cataplexy.

6. Pitolisant for use according to claim 4 where said patient suffers from narcolepsy without cataplexy.

7. Pitolisant for use according to anyone of the preceding claims, wherein said salt is monhydrochloride.
